Europäisches Patentamt

European Patent Office

Office européen des brevets

(19)

(11) Veröffentlichungsnummer: **0 135 755**
**B1**

# (12) EUROPÄISCHE PATENTSCHRIFT

(45) Veröffentlichungstag der Patentschrift:
04.02.87

(21) Anmeldenummer: 84109423.8

(22) Anmeldetag: 08.08.84

(51) Int. Cl.⁴: **A 61 F 2/36**

(54) **Schaft für eine Hüftgelenkprothese.**

(30) Priorität: 30.08.84 DE 3331162

(43) Veröffentlichungstag der Anmeldung:
03.04.85 Patentblatt 85/14

(45) Bekanntmachung des Hinweises auf die Patenterteilung:
04.02.87 Patentblatt 87/6

(84) Benannte Vertragsstaaten:
AT BE CH DE FR GB IT LI NL SE

(56) Entgegenhaltungen:
EP-A-0 027 160
EP-A-0 058 745
EP-A-0 093 378
DE-A-2 356 464
DE-A-2 627 589
DE-B-2 537 807
DE-U-8 213 101
US-A-2 719 522
US-A-2 781 758

(73) Patentinhaber: **Protek AG, Stadtbachstrasse 64, CH-3001 Bern (CH)**

(72) Erfinder: **Spotorno, Lorenzo, Dr., Ospedale Riuniti, I-17024 Finale Ligure (IT)**

(74) Vertreter: **Lusuardi, Werther Giovanni, Dr., Patentanwalt Stockerstrasse 8, CH- 8002 Zürich (CH)**

EP 0 135 755 B1

## Beschreibung

Die Erfindung betrifft eine kragenlose Femurkomponente einer Hüftgelenkprothese mit einem geraden, vom distalen Ende her sich allseitig konisch erweiternden Schaft, wobei der proximale Teil des Schaftes eine, von einer in der proximalen Hälfte des Schaftes liegenden Unstetigkeit ausgehende, nach proximal gerichtete zusätzliche konische Erweiterung der nach anterior bzw. posterior gerichteten Seiten des Schaftes aufweist.

Es ist eine Femurkomponente einer Hüftgelenkprothese gemäss dem ersten Teil des Patentanspruchs 1 bekannt (US-A-2'719'522), bei welcher der sich regelmässig von distal nach proximal erweiternde kegelstumpfförmige Schaft eine zusätzliche, konische Erweiterung mit einem annähernd rechteckigen Querschnitt im proximalen Schaftbereich aufweist. Eine solche dem proximalen Markraum des Femur nur grob angepasste Aussenform ergibt jedoch keine optimale Verankerung im proximalen Bereich, wie sie insbesondere für zementlos zu implantierende Prothesenschäfte von Vorteil ist. Es hat sich überdies in den letzten Jahren gezeigt, dass sich Hüftgelenkprothesen im Laufe der Zeit setzen, d.h. tiefer in den Knochen einsinken. Dabei ist es wichtig, dass die Prothese sich bei diesem Einsinken sofort wieder verklemmt und verkeilt, damit an der Grenzfläche- von Prothese oder Zementbett- zum Knochen keine Mikrobewegungen auftreten, die bekanntlich zu einem Knochenbau führen können.

Der in der US-A-2'719'522 beschriebene Prothesenschaft ermöglicht aber nur eine Wiederverklemmung bei einem axialen Einsinken des Prothesenschaftes.

Es hat sich nun jedoch gezeigt, dass ein Einsinken des Prothesenschaftes nicht nur in Richtung seiner Längsachse stattfindet, sondern dass aufgrund des von der am Gelenkkopf der Femurkomponente angreifenden Belastung ausgeübten Momentes bezüglich der Längsachse ein nach medial gerichtetes Einsinken besonders im proximalen Bereich des Schaftes erfolgt.

Schliesslich ist aus der US-A-2'781'758 ein Hüftprothesenschaft bekannt, der sich bereits in seiner distalen, unteren Hälfte konisch verbreitert und somit lediglich eine Führungsfunktion ausüben kann. Ein Absinken des Prothesenschaftes im Markraum kann damit nicht verhindert werden. Konsequenterweise ist diese bekannte Femurkomponente mit einem Kragen ausgestattet, der die dauerhafte Verankerung garantiert.

Hier will die Erfindung Abhilfe schaffen. Die Erfindung, wie sie in den Ansprüchen gekennzeichnet ist, löst die Aufgabe, eine Femurkomponente einer Hüftgelenkprothese zu schaffen, welche durch ihre konstruktive Ausgestaltung als Mehrfachkonus eine vorwiegend im proximalen Teil des Schaftes erfolgende Verankerung ermöglicht und insbesondere auch für die Setzbewegungen des Prothesenschaftes in medialer Richtung eine möglichst rasch wirkende neue Fixierung sicherstellt. Diese Aufgabe wird durch die Femurkomponente gemäss Anspruch 1 gelöst.

Die durch die Erfindung erreichten Vorteile sind im wesentlichen darin zu sehen, dass sich der Schaft - ebenso wie bei Setzbewegungen in Richtung der Längsachse - beim Nachgeben in medialer Richtung sofort wieder verkeilt und verklemmt und dass durch die von lateral nach medial verlaufende Schaftverjüngung eine optimale Übertragung von Torsionskräften ermöglicht wird.

Die Aufnahme von Torsionskräften im proximalen Schaftbereich wird weiter verbessert indem man den Höhenbereich der Seitenflanken proximal der Unstetigkeit mit rillenartigen Vertiefungen versieht, die gemäß Anspruch 2 im wesentlichen in Richtung der Längsmittelachse des Schaftes verlaufen; es ist jedoch unter Umständen von Vorteil, wenn die rillenförmigen Vertiefungen im wesentlichen in Richtung der Hauptbälkchenzüge (Knochentrabekel) der den Schaft umschliessenden Spongiosaarchitektur verlaufen. Die ausgeprägte axiale Rippenstruktur ermöglicht, die Spongiosaarchitektur des Femur in die Formschlüssigkeit des Prothesenschaftes mit einzubeziehen.

Als Materialien für den neuen Schaft sind alle für Prothesenschäfte von Endoprothesen üblichen Werkstoffe, insbesondere Titanlegierungen geeignet.

Im folgenden wird die Erfindung anhand eines Ausführungsbeispiels im Zusammenhang mit der Zeichnung näher erläutert.

Fig.1 zeigt den neuen Prothesenschaft in einer Aufsicht auf das Schaftblatt;,

Fig.2 ist eine Ansicht von Fig.1 von links; und

Fig.3 gibt den Schnitt III-III von Fig.1 wieder.

Vom distalen Ende 1 aus erweitert sich der blattartige Schaft 2 zunächst konisch symmetrisch zu einer Längsmittelachse 3. Die laterale Schmalseite 4 endet in einer mindestens nahezu horizontalen Schulter 5, die den Übergang zum Prothesenhals 6 bildet. In dieser Schulter 5 ist, konzentrisch mit der Längsmittelachse 3 des Schaftes 2, eine Vertiefung 12 für den Einsatz eines Einschlaginstruments vorgesehen.

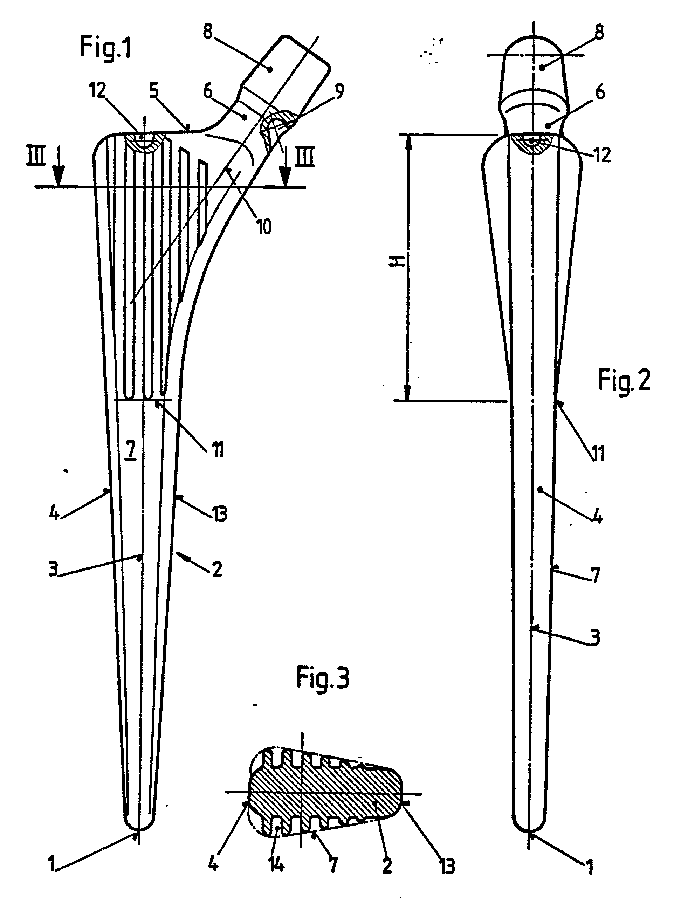

Der sich erweiternde Konus der medialen Schmalseite 13 der Seitenflanken oder Blattseiten 7 geht in einen Kreisbogen über, der stufenlos in den Prothesenhals 6 mündet. Dieser trägt einen Zapfen 8, auf den der nicht dargestellte Gelenkkopf aufgesteckt wird. Der Schenkelhals-Winkel (CCD-Winkel) beträgt 145°. An der Unterseite des Prothesenhalses 6 ist eine weitere Vertiefung 9 vorgesehen; in diese kann gegebenenfalls ein Ausschlaginstrument eingesetzt werden. Aus Fig.2 und 3 ist ersichtlich, dass die Längsachse 3 und die Achse 10 des Prothesenhalses 6 eine Mittelebene des Schaftes 2 definieren, die gleichzeitig eine Symmetrieebene des Schaftes 2 ist.

Am distalen Ende 1 sind die Schmalseiten 4 und 13 und die Blattseiten 7 durch einen halbellipsoidförmigen Übergang abgeschlossen.

Wie Fig.2 erkennen lässt, erweitern sich nicht nur die Blattseiten 7, sondern die Schmalseiten 4 bzw. 13 vom distalen Ende 1 aus konisch, wenn auch der Öffnungswinkel relativ klein und beispielsweise 0,5 -3° gegen die Vertikale beträgt.

Wie Fig.2 verdeutlicht, besitzen die konischen Erweiterungen der Blattseiten 7, die bei eingesetzter Prothese nach anterior bzw. posterior zeigen, eine Unstetigkeit 11. Von dieser nach proximal ist der von den Blattseiten 7 mit der Mittel-oder Symmetrieebene des Schaftes gebildete Öffnungs- oder Konuswinkel vergrössert; während dieser Öffnungs- oder Konuswinkel im distalen Bereich des gezeigten Beispiels etwa 1,5° beträgt, liegt sein Wert im proximalen Bereich zwischen 4° und 10°, vorzugsweise zwischen 6° und 8°; im gezeigten Beispiel ist dieser Winkel etwa 7°.

Der auf diese Weise verbreiterte und verstärkte proximale Bereich des Schaftes 2 bildet im Querschnitt (Fig.3) ein gleichseitiges Trapez, dessen Ecken abgerundet sind. Von der relativ breiten, lateralen Seite 4 verjüngt sich, symmetrisch zur Mittelebene des Schaftes, der Querschnitt dabei erfindungsgemäss zur medialen Schmalseite 13 hin, so dass die Blattseiten 7 einen dritten Konus bilden. Sein Winkel gegen die Mittelebene kann dabei zwischen 5° und 20°, vorzugsweise 10°-15°, betragen.

Wie bereits geschildert, ermöglicht dieser Konus eine rasche Verkeilung, wenn sich die Prothese einige Zeit nach der Implantation "setzt", d.h. mit einer Nickbewegung des Gelenkkopfes eventuell auftretenden Reaktionen des Knochens auf den Fremdkörper, den die Prothese bildet, nachgibt.

Der proximale Höhenbereich H der Blattseiten oder Seitenflanken 7 ist mit rillenartigen Vertiefungen 14 (Fig.3) versehen, die so angeordnet sind, dass ihre Talsohle im wesentlichen parallel zur Einschlagrichtung des Implantats verläuft, die mit der Richtung der Längsmittelachse 3 übereinstimmt.

Die rillenartigen Vertiefungen 14, die eine minimale Tiefe von 1 mm, vorzugsweise von 2 mm, haben können, haben die Aufgabe, möglichst weit proximal eine Verankerung und Fixierung des Schaftes vor allem bei einer zementfrei zu implantierenden Prothese zu gewährleisten und dabei in erster Linie die auf den Schaft 2 wirkenden Biege-und Torsionskräfte auf den Knochen zu übertragen. Die Herstellung der Vertiefungen 14 kann bei metallischen Schaften beispielsweise durch Fräsen mit Hilfe eines Formfräsers, der das Profil der Vertiefungen 14 hat, vorgenommen werden.

Die Abstände der rillenartigen Vertiefungen 14 sollten 2 bis 8 mm, vorzugsweise 3 bis 6 mm betragen. Durch diese Strukturierung ergibt sich eine erhebliche Vergrosserung der mit dem Knochen in Kontakt tretenden Oberfläche des Prothesenschaftes und damit eine innigere Verankerung, welche zusammen mit der mehrfachkonusförmigen Gestalt eine optimale Verankerung im proximalen Bereich des Femur garantiert.

Die Oberflächenvergrösserung beträgt vorteilhafterweise das 1,3 bis 2,7-fache, vorzugsweise das 1,6 bis 2,4-fache der unstrukturierten Oberfläche.

Im distalen Bereich der Femurkomponente wurde bewusst auf eine Oberflächenstrukturierung verzichtet, um die Sekundärfixation (durch Einsinken) nicht zu hindern.


**Patentansprüche**

1. Kragenlose Femurkomponente einer Hüftgelenkprothese mit einem geraden vom distalen Ende (1) her sich allseitig konisch erweiternden Schaft (2), wobei der proximale Teil des Schaftes (2) eine, von einer in der proximalen Hälfte des Schaftes (2) liegenden Unstetigkeit (11) ausgehende, nach proximal gerichtete zusätzliche konische Erweiterung der nach anterior bzw. posterior gerichteten Seiten (7) des Schaftes (2) aufweist, dadurch gekennzeichnet, daß der proximale Teil des blattartigen Schaftes (2) eine, von der lateralen Seite (4) gegen die mediale Seite (13) des Schaftes (2) hin mindestens im Höhenbereich (H) proximal der Unstetigkeit (11) sich erstreckende konische Verjüngung aufweist und daß der Höhenbereich (H) der nach anterior bzw. posterior gerichteten Blattseiten (7) proximal der Unstetigkeit (11) mit rillenartigen Vertiefungen (14) versehen ist.

2. Femurkomponente nach Anspruch 1, dadurch gekennzeichnet, dass die Vertiefungen (14) im wesentlichen in Richtung der Längsmittelachse (3) des Schaftes (2) verlaufen.

3. Femurkomponente nach Anspruch 1, dadurch gekennzeichnet, dass die rillenförmigen Vertiefungen (14) im wesentlichen in Richtung der Hauptbälkchenzüge der den Schaft (2) umschliessenden Spongiosaarchitektur des Femur verlaufen.

4. Femurkomponente nach einem der Ansprüche 1 bis 3, dadurch gekennzeichnet, dass der Konuswinkel der Blattseiten (7) des Schaftes (2) im proximalen Bereich zwischen 4° und 10° vorzugsweise zwischen 6° und 8°, beträgt.

5. Femurkomponente nach einem der Anspruche 1 bis 4, dadurch gekennzeichnet, dass die Verjüngung von lateral nach medial einen Konuswinkel zwischen 5° und 20°, vorzugsweise zwischen 10° und 15°, aufweist.

6. Femurkomponente nach einem der Ansprüche 1 bis 5, dadurch gekennzeichnet, dass die lateral gelegenen rillenartigen Vertiefungen (14) am proximalen Konusende eine minimale Tiefe von 1 mm, vorzugsweise von 2 mm, aufweisen.

7. Femurkomponente nach einem der Ansprüche 1 bis 6, dadurch gekennzeichnet, dass die Abstände der rillenartigen Vertiefungen (14) 2 bis 8 mm, vorzugsweise 3 bis 6 mm betragen.

8. Femurkomponente nach einem der Ansprüche 1 bis 7, dadurch gekennzeichnet, dass die Oberfläche des proximalen Teils des Schaftes (2) durch die rillenartigen Vertiefungen (14) um das 1,3-bis 2,7-fache, vorzugsweise um das 1,6- bis 2,4-fache vergrössert ist.

9. Femurkomponente nach einem der Ansprüche 1 bis 8, dadurch gekennzeichnet, dass der Schaftquerschnitt im proximalen Bereich trapezoid, vorzugsweise in Form eines gleichseitigen Trapezes, ausgebildet ist.

10. Femurkomponente nach einem der Ansprüche 1 bis 9, dadurch gekennzeichnet, dass der distale Teil des Schaftes eine glatte Oberfläche aufweist.

**Claims**

1. Collarless femur component of a hip joint prosthesis with a straight shaft (2) continuously widening conically from the distal end (1), whereby the proximal part of said shaft (2) shows an additional, proximally directed conical enlargement of the anterior, respectively posterior sides (7) of said shaft (2), said enlargement starting from a discontinuity (11) located in the proximal half of said shaft (2), characterized in that the proximal part of the blade-like shaft (2) shows a narrowing conical structure extending from the lateral side (4) to the medial side (13) of said shaft (2) at least over the range of height (H) proximal to said discontinuity (11) and that said range of height (H) of said anterior, respectively posterior blade sides (7) is provided with groove-like recesses (14) proximally to said discontinuity (11).

2. Femur component according to claim 1, characterized in that said recesses (14) extend essentially in the direction of the longitudinal center axis (3) of said shaft (2).

3. Femur component according to claim 1, characterized in that said groove-like recesses (14) extend essentially in the direction of the main bar courses of the spongiosa structure of the femur surrounding said shaft (2).

4. Femur component according to one of the claims 1 to 3, characterized in that the cone angle of said blade sides (7) of said shaft (2) is between 4° and 10°, preferably between 6° and 8°, measured in the proximal range.

5. Femur component according to one of the claims 1 to 4, characterized in that said conical structure narrowing from lateral to medial has a cone angle of between 5° and 20°, preferably of between 10° and 15°.

6. Femur component according to one of the claims 1 to 5, characterized in that the laterally located groove-like recesses (14) are formed with a minimum depth of 1 mm, preferably of 2 mm, at the proximal end of said conical enlargement.

7. Femur component according to one of the claims 1 to 6, characterized in that the distances between said groove-like recesses (14) measure 2 to 8 mm, preferably 3 to 6 mm.

8. Femur component according to one of the claims 1 to 7, characterized in that the surface of the proximal part of said shaft (2) is enlarged by said groove-like recesses (14) by a factor of between 1,3 and 2,7, preferably of between 1,6 and 2,4.

9. Femur component according to one of the claims 1 to 8, characterized in that the cross-sectional configuration in the proximal range of said shaft (2) is in the shape of a trapezoid, preferably in the shape of an equilateral trapezoid.

10. Femur component according to one of the claims 1 to 9, characterized in that the distal part of said shaft (2) has a smooth surface.

**Revendications**

1. Tige fémorale sans collet d'une prothèse de hanche avec une tige (2) droite s'élargissant coniquement du bout distal (1) dans toutes directions, la partie proximale de la tige (2) ayant un élargissement conique supplémentaire des faces (7) antérieures et postérieures de la tige (2) s'élargissant en direction proximale à partir d'une discontinuité située dans la moitié proximale de la tige (2), caractérisée en ce que la partie proximale de la tige (2) en forme de lame comporte une conicité se projettant de la partie latérale (4) à la partie médiale (13) de la tige (2) au moins dans la région de hauteur (H) proximale de la discontinuité (11) et que la région de hauteur (H) des faces antérieures et postérieures (7) de la tige (2) est pourvue de cavités (14) en forme de rainures situées proximalement de la discontinuité (11).

2. Tige fémorale selon la revendication 1, caractérisée en ce que les cavités (14) courent essentiellement en direction de la axe centrale longitudinale (3) de la tige (2).

3. Tige fémorale selon la revendication 1, caractérisée en ce que les cavités (14) en forme de rainures courent essentiellement en direction de l'architecture lamellaire du tissu spongieux du femur entourant la tige (2).

4. Tige fémorale selon une des revendications 1 à 3, caractérisée en ce que l'angle du cône des faces (7) antérieures et postérieures de la tige (2) est compris entre 4° et 10°, préférablement entre 6° et 8°, dans la région proximale.

5. Tige fémorale selon une des revendications 1 à 4, caractérisée en ce que la conicité se projettant de la partie latérale (4) à la partie médiale (13) de la tige (2) comporte un angle de cône entre 5° et 20°, préférablement entre 10° et 15°.

6. Tige fémorale selon une des revendications 1 à 5, caractérisée en ce que les cavités (14) en forme de rainures, situées latéralement, ont une profondeur minimale de 1 mm, préférablement de 2 mm à l'éxtrémité proximale du cône.

7. Tige fémorale selon une des revendications 1 à 6, caractérisée en ce que la distance entre les cavités (14) en forme de rainures est de 2 à 8 mm, préférablement de 3 à 6 mm.

8. Tige fémorale selon une des revendications 1 à 7, caractérisée en ce que la surface de la partie proximale de la tige (2) est augmentée par le facteur 1,3 à 2,7, préférablement par le facteur 1,6 à 2,4.

9. Tige fémorale selon une des revendications 1 à 8, caractérisée en ce que la section de la tige (2) dans la région proximale est trapézoïdale, préférablement en forme d'un trapéze équilatéral.

10. Tige fémorale selon une des revendications 1 à 9, caractérisée en ce que la partie distale de la tige (2) dispose d'une surface lisse.

Fig.1

Fig.2

Fig.3